# EUROPÄISCHE PATENTSCHRIFT

(11) **EP 3 808 382 B1**
(45) Veröffentlichungstag und Bekanntmachung des Hinweises auf die Patenterteilung: **18.10.2023**
(21) Anmeldenummer: 20202040.0
(22) Anmeldetag: 15.10.2020
(51) Int. Cl.: A61L 2/07, B65B 55/04, B65B 55/10, B67C 7/00, A61L 2/18, A61L 2/20, A61L 2/22

(54) **STERILISATION VON BEHÄLTERN IN GETRÄNKEABFÜLLANLAGEN**
STERILIZATION OF CONTAINERS IN BEVERAGE FILLING SYSTEMS
STÉRILISATION DE RÉCIPIENTS DANS LES SYSTÈMES DE REMPLISSAGE DE BOISSONS

(30) Priorität: 15.10.2019 DE 102019127711
(43) Veröffentlichungstag der Anmeldung: 21.04.2021
(73) Patentinhaber: KRONES AG, 93073 Neutraubling (DE)
(72) Erfinder: Soellner, Juergen, 93073 Neutraubling (DE); Hausladen, Josef, 93073 Neutraubling (DE); Soellner-Wein, Gertrud, 93073 Neutraubling (DE)
(74) Vertreter: Nordmeyer, Philipp Werner

(56) Entgegenhaltungen:
- EP-A1- 1 607 106
- EP-A1- 2 653 396
- EP-B1- 2 653 396
- DE-A1-102005 009 875
- DE-A1-102006 036 462
- DE-A1-102008 031 369
- DE-A1-102017 212 337
- DE-A1-102017 215 200
- DE-B4-102017 212 337
- JP-A- 2014 080 207

## Beschreibung

### Technisches Gebiet

Die vorliegende Erfindung betrifft eine Vorrichtung und ein Verfahren zur Behandlung von Verpackungsmitteln mit einem Behandlungsmittel, insbesondere zur Sterilisation von Behältern in einer Getränkeabfüllanlage.

### Stand der Technik

Bei der Abfüllung von Behältern mit Lebensmitteln, beispielsweise Fruchtsäften, Softdrinks und dergleichen, werden aus hygienischen und gesundheitlichen Gründen eine hohe Reinheit und ein hoher Sterilisationsgrad angestrebt. Zu diesem Zweck werden die Behälter, beispielsweise Glasflaschen oder PET-Flaschen, vor dem Abfüllen sterilisiert.

Die Sterilisation kann mittels eines Gemisches aus Wasserdampf und einem Desinfektionsmittel erfolgen. So ist es bekannt, zur Behälterentkeimung auf Aseptikanlagen flüssige Peressigsäure zu verwenden. Peressigsäure wird dazu auf eine Anwendungskonzentration von etwa 2500ppm verdünnt, kalt zu einer Düse am Behandlungsaggregat gepumpt und dort in einer Zweistoffdüse mit Dampf gemischt und direkt als Dampfnebel in den Behälter eingeleitet. Durch die Mischung mit Dampf wird die Peressigsäure erhitzt, beispielsweise auf etwa 70°C, und verdünnt.

Eine Variante und Weiterentwicklung dieses Verfahrens, das insbesondere für die Anwendung von Kunststoffbehältern optimiert ist, geht aus der EP 2 653 396 A1 hervor.

Für viele bekannte Keime wie "Bacillus Atrophaeus" ist bei diesem Verfahren die Abtötungskinetik ausreichend. Es treten jedoch zunehmend Keime auf, die gegenüber der verwendeten Form von Peressigsäure, im Weiteren auch als "PAA" bezeichnet, erhebliche Resistenzen aufweisen. Im speziellen wird hierbei eine Art mit dem Namen "Phaenibacillus chibensis/ favisporus" zunehmend zum Problem, speziell im asiatischen Raum. Dieser Keim, im Weiteren auch als "PC" bezeichnet, ist gegenüber PAA sehr resistent.

Zur Sterilisation in der Verpackungstechnik wird PAA zumeist in der Form eines in einem chemischen Gleichgewicht stehenden Gemischs aus Peressigsäure, Essigsäure und HzOz (Wasserstoffperoxid) angewendet. Die Mischungsverhältnisse dieser drei Bestandteile sind dabei nicht beliebig einstellbar, da die Mischung immer wieder einem von mehreren stabilen Gleichgewichtszuständen zustrebt.

Es ist bekannt, dass die Abtötungswirkung von PAA mit einem geringeren Anteil HzOz bei PC deutlich effizienter ist als in einem Standardgleichgewicht. Eine Reduzierung des H₂O₂-Anteils kann dadurch erreicht werden, dass dem PAA-Gemisch Katalase zugeführt wird, wie es aus der JP 2014-080207 A2 bekannt ist.

Solche Verfahren bedürfen jedoch der Verwendung vergleichsweise teurer Katalase-Enzyme, die zudem mit einem gewissen Vorlauf dosiert werden müssen, um die nötige Reaktionszeit zu gewährleisten. Das dadurch entstandene PAA-Gemisch ist nicht lagerfähig, da sich das ursprüngliche Gleichgewicht wieder einstellt.

Die EP 1 607 106 A1 beschreibt eine Gefäßbehandlungsmaschine gemäß dem Oberbegriff des Anspruchs 1 zur Sterilisation von Behältern mittels H₂O₂. Die DE 10 2008 031 369 A1 beschreibt ein Verfahren und eine Vorrichtung zur Behandlung von Kunststoff-Einwegbehältern. Die DE 10 2017 215 200 A1 beschreibt einen Verdampfer mit mäandrierendem Strömungskanalsystem. Die EP 2 653 396 A1 betrifft die Sterilisation von Verpackungsbehältern.

### Darstellung der Erfindung

Ausgehend von dem bekannten Stand der Technik ist es eine Aufgabe der vorliegenden Erfindung, eine verbesserte Vorrichtung und ein verbessertes Verfahren zur Behandlung von Verpackungsmitteln, insbesondere zur Sterilisation von Behältern in einer Getränkeabfüllanlage, mit einem Behandlungsmittel anzugeben.

Die Aufgabe wird durch eine Vorrichtung mit den Merkmalen des Anspruchs 1 sowie einem Verfahren mit den Merkmalen des nebengeordneten Verfahrensanspruchs gelöst. Vorteilhafte Weiterbildungen folgen aus den Unteransprüchen, der folgenden Darstellung der Erfindung sowie der Beschreibung bevorzugter Ausführungsbeispiele.

Die Vorrichtung dient der Behandlung von Verpackungsmitteln, die bevorzugt in der Lebensmittelindustrie verwendet werden. Die Behandlung umfasst insbesondere eine Desinfektion und/oder Sterilisation zur Abtötung von Keimen. Besonders bevorzugt findet die Behandlung vor dem Abfüllen flüssiger Lebensmittel statt, als Station oder in Zusammenarbeit mit einer Abfüllanlage zum Befüllen eines Behälters mit Getränken, Saucen oder dergleichen, wie beispielsweise Wasser, karbonisiert oder nicht-karbonisiert, Softdrinks, Bier, Fruchtsäften, Smoothies oder Mischgetränken.

Wenn Bezeichnungen wie Behälter", "Behandlungsorgan" und andere hierin im Singular verwendet werden, geschieht dies in erster Linie der sprachlichen Einfachheit halber. Der Plural ist mitumfasst, sofern dieser nicht ausdrücklich oder technisch ausgeschlossen ist.

Die Vorrichtung weist zumindest ein Behandlungsorgan auf, das eingerichtet ist, um das Verpackungsmittel zumindest abschnittsweise mit dem Behandlungsmittel zu beaufschlagen.

Ferner ist eine Behandlungsmittelzuleitung vorgesehen, die eingerichtet ist, um das Behandlungsmittel dem Behandlungsorgan zuzuleiten. Erfindungsgemäß weist die Vorrichtung eine Katalysatoreinrichtung auf, die in der Behandlungsmittelzuleitung angeordnet und eingerichtet ist, um vom Behandlungsmittel durchströmt zu werden und das Behandlungsmittel chemisch zu verändern. Die chemische Veränderung dient hierbei der Verbesserung oder Optimierung der Wirksamkeit des Behandlungsmittels. Dies wird vorzugsweise dadurch erreicht, dass Anteile von Komponenten des Behandlungsmittels, sofern es aus mehreren Komponenten besteht, von der Katalysatoreinrichtung relativ zueinander verändert werden. Dies kann auch das Aufheben eines chemischen Gleichgewichtzustands umfassen, da die hierin dargelegte Vorrichtung imstande ist, Behandlungsmittel auch in instabilen chemischen Zuständen anzuwenden.

Ein wichtiger technischer Beitrag der erfindungsgemäßen Behandlungsvorrichtung besteht darin, dass zur Optimierung oder Steigerung der Wirksamkeit des Behandlungsmittels auf Zugabe teurer Enzyme, wie etwa Katalase, oder anderer chemischer Stoffe verzichtet werden kann. Die Wirksamkeitssteigerung wird somit ressourcen- und kostenschonend erzielt. Es kommt hinzu, dass die Optimierung der Wirksamkeit mit nur geringen baulichen Veränderungen herkömmlicher Systeme erzielbar ist, nämlich allein durch Installation der Katalysatoreinrichtung. Damit ist eine etwaige Nachrüstung bestehender Anlagen kostengünstig und zeitsparend durchführbar.

Indem die Katalysatoreinrichtung in der Behandlungsmittelzuleitung, somit unmittelbar vor dem Aufbringen auf das Verpackungsmittel, installiert ist, benötigt die Herstellung des verbesserten Behandlungsmittels keinen oder nur einen unerheblichen Vorlauf. Damit sind auch jene Formen der Wirksamkeitssteigerung implementierbar, die auf einem chemisch instabilen Zustand des Behandlungsmittels beruhen.

Vorzugsweise weist die Katalysatoreinrichtung eine Katalysatorkammer gefüllt mit einem Katalysatormaterial, beispielsweise in Form von Kügelchen, auf. Das Katalysatormaterial ist im Wesentlichen inert, d.h. chemisch stabil, gegenüber dem Behandlungsmittel. Vorzugsweise umfasst das Katalysatormaterial Platinoxid und/oder Mangandioxid. Auf diese Weise kann die Katalysatoreinrichtung auf baulich einfache, preiswerte und zuverlässige Weise realisiert werden.

Vorzugsweise weist die Katalysatoreinrichtung einen Filter, beispielsweise umfassend ein Filtervlies, auf, der vom Behandlungsmittel durchströmbar in der Katalysatorkammer angebracht bzw. verspannt ist, wodurch das Behandlungsmittel baulich integriert von etwaigen Verunreinigungen befreit werden kann.

Vorzugsweise weist die Vorrichtung ferner eine Dampfzuleitung auf, die eingerichtet ist, um Dampf, vorzugsweise Heißdampf, dem Behandlungsorgan zuzuleiten. Zu diesem Zweck weist das Behandlungsorgan etwa einen Mischraum in Fluidverbindung mit der Behandlungsmittelzuleitung und der Dampfzuleitung auf, der eingerichtet ist, um das Behandlungsmittel durch den Dampf zu verdünnen, vorzugsweise ferner zu temperieren, und das Gemisch über eine Düse auf das Verpackungsmittel abzugeben. Auf diese Weise kann das Behandlungsmittel je nach Anwendung geeignet verdünnt und somit dosiert werden. Zudem kann das Behandlungsmittel kalt zugeführt und durch den Dampf auf synergetische Weise temperiert, insbesondere erhitzt, werden. Das an sich bekannte Anwendungskonzept mittels Heißdampf und unter Anwendung einer Zweistoffdüse kann weitestgehend beibehalten werden, wobei lediglich der Zweig zur Zufuhr des (kalten) Behandlungsmittels um eine Katalysatoreinrichtung ergänzt wird. Die Herstellung des Behandlungsmittels sowie die Erzeugung des Heißdampfes, die Zusammenführung, etwaige Zerstäubung und Beaufschlagung der Medien kann im Wesentlichen unverändert weiter genutzt werden, wodurch eine etwaige Nachrüstung bestehender Anlagen besonders kostengünstig, ressourcenschonend und zeitsparend durchführbar ist.

Gemäß der Erfindung ist das Behandlungsmittel ein Gemisch aus Peressigsäure, Essigsäure und Wasserstoffperoxid. Das Gemisch befindet sich normalerweise in einem stabilen Gleichgewicht. Ein solches Peressigsäure-Gemisch hat sich als besonders wirksam zur Abtötung vieler bekannter Keime, darunter "Bacillus Atrophaeus", herausgestellt. Um die Wirkung auch auf andere Keime wie beispielsweise "Phaenibacillus chibensis/ favisporus" auszudehnen, ist der hierin beschriebene Katalysator sehr gut geeignet, da er den Wasserstoffperoxid-Anteil im Gemisch reduziert und so die Wirksamkeit des Behandlungsmittels erhöht. Eine Reduzierung des HzOz-Anteils auf null ist hierbei nicht unbedingt erforderlich, da bereits eine Erhöhung des relativen Anteils an Peressigsäure zu guten Abtötungsergebnissen führt, insbesondere hinsichtlich vermehrt im asiatischen Raum auftretendem "Phaenibacillus chibensis/ favisporus".

Die oben genannte Aufgabe wird ferner durch ein Verfahren zur Behandlung von Verpackungsmitteln, insbesondere zur Sterilisation von Behältern in einer Getränkeabfüllanlage, mit einem Behandlungsmittel gelöst, wobei das Verfahren umfasst: Bereitstellen eines Behandlungsmittels, vorzugsweise Sterilisationsmittels; Durchleiten des Behandlungsmittels durch eine Katalysatoreinrichtung, wodurch das Behandlungsmittel chemisch verändert wird; Beaufschlagen des Verpackungsmittels mit dem durch die Katalysatoreinrichtung chemisch veränderten Behandlungsmittel.

Die Merkmale, technischen Wirkungen, Vorteile sowie Ausführungsbeispiele, die in Bezug auf die Vorrichtung beschrieben wurden, gelten analog für das Verfahren, und umgekehrt.

So besteht das Behandlungsmittel vorzugsweise aus mehreren Komponenten, wobei die Katalysatoreinrichtung aus den oben genannten Gründen vorzugsweise den Anteil einer oder mehrerer Komponenten relativ zueinander verändert.

Vorzugsweise befinden sich die Anteile der Komponenten in einem chemischen Gleichgewicht, wobei die Katalysatoreinrichtung aus den oben genannten Gründen in diesem Fall das Behandlungsmittel vorzugsweise aus dem chemischen Gleichgewicht, d.h. in einen instabilen Zustand, bringt.

Vorzugsweise weist die Katalysatoreinrichtung aus den oben genannten Gründen eine Katalysatorkammer gefüllt mit einem Katalysatormaterial, vorzugsweise in Form von Kügelchen, das inert gegenüber dem Behandlungsmittel ist, auf. Vorzugsweise umfasst das Katalysatormaterial Platinoxid und/oder Mangandioxid.

Gemäß der Erfindung ist oder umfasst das Behandlungsmittel aus den oben genannten Gründen ein Gemisch aus Peressigsäure, Essigsäure und Wasserstoffperoxid, vorzugsweise im Verhältnis eines stabilen Gleichgewichts.

Ferner reduziert die Katalysatoreinrichtung aus den oben genannten Gründen den Wasserstoffperoxid-Anteil im Gemisch.

Vorzugsweise wird aus den oben genannten Gründen ferner Dampf, vorzugsweise Heißdampf, bereitgestellt, das dem durch die Katalysatoreinrichtung chemisch veränderten Behandlungsmittel zugemischt wird, wodurch das Behandlungsmittel verdünnt, vorzugsweise temperiert, wird, bevor das Gemisch auf das Verpackungsmittel abgegeben wird.

Vorzugsweise ist das Verpackungsmittel ein Behälter, wobei das durch die Katalysatoreinrichtung chemisch veränderte Behandlungsmittel in das Behälterinnere eingebracht, vorzugsweise eingesprüht, wird. Als Behälter kommen beispielsweise Glasflaschen oder PET-Flaschen in Betracht.

Weitere Vorteile und Merkmale der vorliegenden Erfindung sind aus der folgenden Beschreibung bevorzugter Ausführungsbeispiele ersichtlich.

Die folgende Beschreibung bevorzugter Ausführungsbeispiele erfolgt dabei mit Bezug auf die begleitenden Zeichnungen.

### Kurze Beschreibung der Figuren

Bevorzugte weitere Ausführungsformen der Erfindung werden durch die nachfolgende Beschreibung der Figuren näher erläutert. Dabei zeigen:
Figur 1 eine schematische Darstellung einer Vorrichtung zur Behandlung von Verpackungsmitteln, insbesondere zur Sterilisation von mit einem Lebensmittel zu befüllenden Behältern;
Figur 2 ein Flussdiagramm zur Darstellung eines Verfahrens zur Behandlung von Verpackungsmitteln, insbesondere zur Sterilisation von mit einem Lebensmittel zu befüllenden Behältern.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren gegebenenfalls mit identischen Bezugszeichen versehen, und auf eine wiederholte Beschreibung dieser Elemente wird teilweise verzichtet, um Redundanzen zu vermeiden.

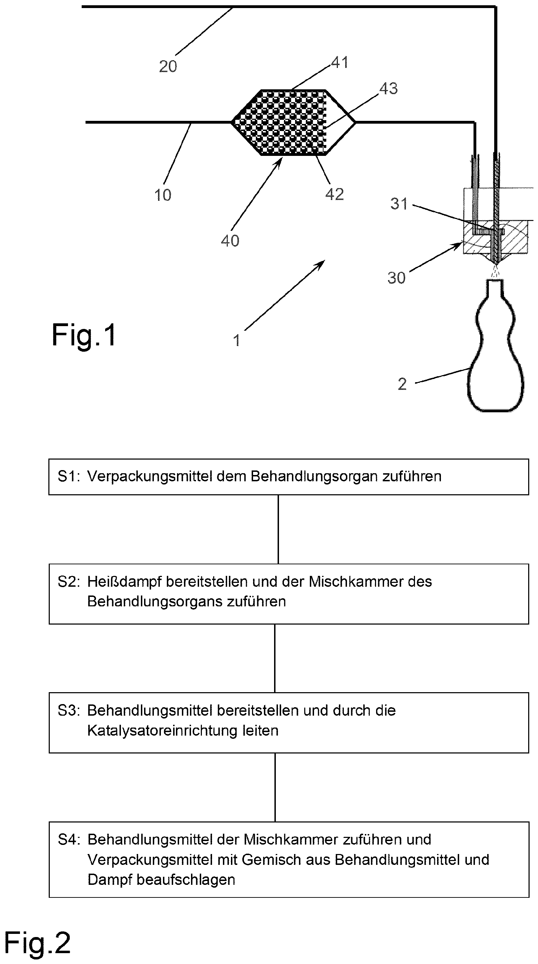

Die Figur 1 ist eine schematische Darstellung einer Vorrichtung 1 zur Behandlung, insbesondere Sterilisation, von Verpackungsmitteln 2. Vorzugsweise handelt es sich bei den Verpackungsmitteln 2 um mit einem Lebensmittel zu befüllende Behälter. Die Vorrichtung 1 wird hierin auch als "Behandlungsvorrichtung" bezeichnet.

Die Vorrichtung 1 weist eine Behandlungsmittelzuleitung 10 für ein fluides Behandlungsmittel zur Behandlung des Behälters 2, insbesondere dessen Innenraum, auf. Das Behandlungsmittel ist vorzugsweise ein Desinfektionsmittel, besonders bevorzugt ein Gemisch aus Peressigsäure, Essigsäure und Wasserstoffperoxid im Verhältnis eines stabilen chemischen Gleichgewichtszustands.

Die Behandlungsmittelzuleitung 10 ist eingerichtet, um das Behandlungsmittel einem Behandlungsorgan 30 zuzuleiten, das eingerichtet ist, um das Verpackungsmittel 2 zumindest abschnittsweise mit dem Behandlungsmittel zu beaufschlagen. Vorzugsweise umfasst das Behandlungsorgan 30 zu diesem Zweck eine oder mehrere Düsen, die das Behandlungsmittel insbesondere in das Innere von Behältern einleiten und dabei gegebenenfalls zerstäuben.

Die Behandlungsmittelzuleitung 10 kann von einem Reservoir oder einer Ringleitung abzweigen. Ferner kann die Behandlungsmittelzuleitung 10 über Einrichtungen zur Überwachung und Steuerung des Durchflusses verfügen, beispielsweise über Ventile, eine Dosiereinrichtung, eine Sensoreinheit und dergleichen, die in der Figur 1 der Übersichtlichkeit halber jedoch nicht dargestellt sind.

Die Behandlungsvorrichtung 1 weist ferner vorzugsweise eine Dampfzuleitung 20 auf, die eingerichtet ist, um Heißdampf dem Behandlungsorgan 30 zuzuleiten. Das Behandlungsorgan 30 weist in diesem Fall einen Mischraum 31 auf, in den das Behandlungsmittel und der Dampf eingeleitet werden, wodurch das Behandlungsorgan 30 als Zweistoffdüse ausgebildet ist, die das Behandlungsmittel und den Dampf mischt und etwa als Dampfnebel dem zu behandelnden Verpackungsmittel 2 zuführt.

Vorzugsweise wird das vorstehend genannte Gemisch aus Peressigsäure, Essigsäure und Wasserstoffperoxid kalt dem Mischraum 31 zugeführt und dort mit dem Heißdampf gemischt, wodurch das Behandlungsmittel verdünnt, vorzugsweise auf eine Anwendungskonzentration von etwa 2500ppm, und gleichzeitig erhitzt wird, vorzugsweise auf etwa 70°C.

In der Behandlungsmittelzuleitung 10 ist eine Katalysatoreinrichtung 40 installiert, die zur chemischen Modifikation des Behandlungsmittels eingerichtet ist, um dessen Wirksamkeit zu verbessern. Besonders bevorzugt dient die Katalysatoreinrichtung 40 dazu, den HzOz-Anteil aus dem genannten Gemisch aus Peressigsäure, Essigsäure und Wasserstoffperoxid zu reduzieren, wodurch die Sterilisationswirkung dieses bevorzugten Behandlungsmittels erweitert wird.

Die Katalysatoreinrichtung 40 ist dem Behandlungsorgan 30, somit dem Mischraum 31, direkt vorgeschaltet, wodurch nur geringe maschinenbauliche Modifikationen zur Nachrüstung einer herkömmlichen Behandlungsvorrichtung erforderlich sind. Ferner ist dadurch gewährleistet, dass sich nach der katalytischen Reaktion bzw. Modifikation, insbesondere Reduzierung des H₂O₂-Anteils, das ursprüngliche Gleichgewicht nicht wieder einstellen kann, bevor das Verpackungsmittel 2 mit dem Behandlungsmittel beaufschlagt wird.

Im Fall von Peressigsäure ist eine Reduzierung des H₂O₂-Anteils auf null nicht unbedingt erforderlich, da bereits eine Erhöhung des relativen Anteils an Peressigsäure zu guten Abtötungsergebnissen insbesondere bezüglich PC führt.

Die Katalysatoreinrichtung 40 umfasst eine von dem Behandlungsmittel durchströmbare Katalysatorkammer 41, die mit einem Katalysatormaterial 42 gefüllt ist. Als Katalysatormaterial 42 wird ein Stoff eingesetzt, der in Flüssigkeit nicht lösbar ist und von den Reaktionspartnern des Behandlungsmittels nicht aufgezehrt wird. Im Fall von Peressigsäure ist das Katalysatormaterial 42 somit inert gegenüber Peressigsäure. Vorzugsweise umfasst das Katalysatormaterial 42 Platinoxid und/oder Mangandioxid. Das Katalysatormaterial 42 kann beispielsweise in Form von Kügelchen in einer Packung in die Katalysatorkammer 41 eingebracht werden. Zusätzlich kann die Katalysatoreinrichtung 40 einen Filter 43, vorzugsweise ein Filtervlies beispielsweise in der Stärke von etwa 0,1mm, aufweisen, der durchströmbar in der Katalysatorkammer 41 angebracht bzw. verspannt ist.

Gemäß einem bevorzugten Behandlungsprozess, veranschaulicht mittels des Flussdiagramms der Figur 2, wird in einem Schritt S1 ein zu behandelndes Verpackungsmittel 2 dem Wirkungsbereich des Behandlungsorgans 30 zugeführt. In einem Schritt S2 wird Heißdampf bereitgestellt und der Mischkammer 31 des Behandlungsorgans 30 zugeführt. In einem Schritt S3 wird das Behandlungsmittel bereitgestellt. Das Behandlungsmittel wird anschließend durch die Katalysatoreinrichtung 40 geleitet, wodurch eine Wirksamkeitssteigerung erzielt wird, vorzugsweise indem das Behandlungsmittel aus einem chemischen Gleichgewicht in einen chemisch instabilen Zustand gebracht wird. Im Fall eines Peressigsäure-Gemischs ist die Katalysatoreinrichtung 40 vorzugsweise eingerichtet, um den HzOz-Anteil zu reduzieren, wodurch die Sterilisationswirkung erweitert wird. Das so optimierte Behandlungsmittel wird in einem Schritt S4 der Mischkammer 31 zugeführt, wo es durch den Heißdampf verdünnt und temperiert wird. Anschließend wird das Verpackungsmittel 2 mit dem Gemisch aus Dampf und Behandlungsmittel beaufschlagt, indem das Behandlungsorgan 30 das Gemisch ausleitet und gegebenenfalls zerstäubt.

Wenngleich die Verfahrensschritte sequentiell beschrieben sind, ist die Durchführung derselben nicht an die oben gewählte Ordnung gebunden. Die Reihenfolge der Verfahrensschritte kann im technisch sinnvollen Rahmen frei gewählt werden, worunter auch eine teilweise oder im Wesentlichen vollständige Parallelausführung fällt.

Ein wichtiger technischer Beitrag der hierin dargelegten Behandlungsvorrichtung 1 sowie des entsprechenden Behandlungsprozesses besteht darin, dass zur Steigerung der Wirksamkeit des Behandlungsmittels auf die Zugabe teurer Enzyme, wie etwa Katalase, oder anderer chemischer Stoffe verzichtet werden kann. Die Wirksamkeitssteigerung wird somit ressourcen- und kostenschonend erzielt.

Es kommt hinzu, dass die Optimierung der Wirksamkeit mit nur geringen baulichen Veränderungen herkömmlicher Systeme erzielbar ist. Insbesondere kann das an sich bekannte Anwendungskonzept mittels Heißdampf und unter Anwendung einer Zweistoffdüse weitestgehend beibehalten werden, wobei lediglich der Zweig zur Zufuhr des kalten Behandlungsmittels um eine Katalysatoreinrichtung erweitert wird. Die Herstellung des Behandlungsmittels sowie die Erzeugung des Heißdampfes, die Zusammenführung, etwaige Zerstäubung und Beaufschlagung der Medien kann im Wesentlichen unverändert weiter genutzt werden. Damit ist eine etwaige Nachrüstung bestehender Anlagen kostengünstig und zeitsparend durchführbar.

### Bezugszeichenliste

- 1: Vorrichtung zur Behandlung von Verpackungsmitteln
- 2: Verpackungsmittel
- 10: Behandlungsmittelzuleitung
- 20: Dampfzuleitung
- 30: Behandlungsorgan
- 31: Mischraum
- 40: Katalysatoreinrichtung
- 41: Katalysatorkammer
- 42: Katalysatormaterial
- 43: Filter

## Patentansprüche

1. Vorrichtung (1) zur Behandlung von Verpackungsmitteln (2), insbesondere zur Sterilisation von Behältern in einer Getränkeabfüllanlage, mit einem Behandlungsmittel, wobei die Vorrichtung (1) aufweist:
ein Behandlungsorgan (30), das eingerichtet ist, um das Verpackungsmittel (2) zumindest abschnittsweise mit dem Behandlungsmittel zu beaufschlagen;
eine Behandlungsmittelzuleitung (10), die eingerichtet ist, um das Behandlungsmittel dem Behandlungsorgan (30) zuzuleiten; und
eine Katalysatoreinrichtung (40), die in der Behandlungsmittelzuleitung (10) angeordnet und eingerichtet ist, um vom Behandlungsmittel durchströmt zu werden und das Behandlungsmittel chemisch zu verändern;
**dadurch gekennzeichnet, dass**
das Behandlungsmittel ein Gemisch aus Peressigsäure, Essigsäure und Wasserstoffperoxid ist und die Katalysatoreinrichtung (40) eingerichtet ist, um den Wasserstoffperoxid-Anteil im Gemisch zu reduzieren.

2. Vorrichtung (1) nach Anspruch 1, **dadurch gekennzeichnet, dass** die Katalysatoreinrichtung (40) eine Katalysatorkammer (41) gefüllt mit einem Katalysatormaterial (42), vorzugsweise in Form von Kügelchen, das inert gegenüber dem Behandlungsmittel ist, aufweist, wobei das Katalysatormaterial (42) vorzugsweise Platinoxid und/oder Mangandioxid umfasst.

3. Vorrichtung (1) nach Anspruch 2, **dadurch gekennzeichnet, dass** die Katalysatoreinrichtung (40) einen Filter (43), vorzugsweise umfassend ein Filtervlies, aufweist, der vom Behandlungsmittel durchströmbar in der Katalysatorkammer (41) angebracht ist.

4. Vorrichtung (1) nach einem der vorigen Ansprüche, **dadurch gekennzeichnet, dass** ferner eine Dampfzuleitung (20) vorgesehen ist, die eingerichtet ist, um Dampf, vorzugsweise Heißdampf, dem Behandlungsorgan (30) zuzuleiten.

5. Vorrichtung (1) nach Anspruch 4, **dadurch gekennzeichnet, dass** das Behandlungsorgan (30) einen Mischraum (31) in Fluidverbindung mit der Behandlungsmittelzuleitung (10) und der Dampfzuleitung (20) aufweist, die eingerichtet ist, um das Behandlungsmittel durch den Dampf zu verdünnen, vorzugsweise zu temperieren, und das Gemisch über eine Düse auf das Verpackungsmittel (2) abzugeben.

6. Vorrichtung (1) nach einem der vorigen Ansprüche, **dadurch gekennzeichnet, dass** das Behandlungsmittel im Verhältnis eines stabilen Gleichgewichtzustands vorliegt.

7. Vorrichtung (1) nach Anspruch 6, **dadurch gekennzeichnet, dass** die Katalysatoreinrichtung (40) eingerichtet ist, um den Wasserstoffperoxid-Anteil im Gemisch zu reduzieren.

8. Verfahren zur Behandlung von Verpackungsmitteln (2), insbesondere zur Sterilisation von Behältern in einer Getränkeabfüllanlage, mit einem Behandlungsmittel, wobei das Verfahren umfasst:
Bereitstellen eines Behandlungsmittels, vorzugsweise Sterilisationsmittels;
Durchleiten des Behandlungsmittels durch eine Katalysatoreinrichtung (40), wodurch das Behandlungsmittel chemisch verändert wird;
Beaufschlagen des Verpackungsmittels (2) mit dem durch die Katalysatoreinrichtung (40) chemisch veränderten Behandlungsmittel;
**dadurch gekennzeichnet, dass**
das Behandlungsmittel ein Gemisch aus Peressigsäure, Essigsäure und Wasserstoffperoxid ist und die Katalysatoreinrichtung den Wasserstoffperoxid-Anteil im Gemisch reduziert.

9. Verfahren nach Anspruch 8, **dadurch gekennzeichnet, dass** das Behandlungsmittel aus mehreren Komponenten besteht und die Katalysatoreinrichtung (40) den Anteil einer oder mehrerer Komponenten verändert.

10. Verfahren nach Anspruch 9, **dadurch gekennzeichnet, dass** sich die Anteile der Komponenten in einem chemischen Gleichgewichtszustand befinden und die Katalysatoreinrichtung (40) das Behandlungsmittel aus dem chemischen Gleichgewichtszustand bringt.

11. Verfahren nach einem der Ansprüche 8 bis 10, **dadurch gekennzeichnet, dass** die Katalysatoreinrichtung (40) eine Katalysatorkammer (41) gefüllt mit einem Katalysatormaterial (42), vorzugsweise in Form von Kügelchen, das inert gegenüber dem Behandlungsmittel ist, aufweist, wobei das Katalysatormaterial (42) vorzugsweise Platinoxid und/oder Mangandioxid umfasst.

12. Verfahren nach einem der Ansprüche 8 bis 11, **dadurch gekennzeichnet, dass** das Behandlungsmittel im Verhältnis eines stabilen Gleichgewichtszustands vorliegt.

13. Verfahren nach Anspruch 12, **dadurch gekennzeichnet, dass** die Katalysatoreinrichtung (40) den Wasserstoffperoxid-Anteil aus dem Gemisch reduziert.

14. Verfahren nach einem der Ansprüche 8 bis 13, **dadurch gekennzeichnet, dass** ferner Dampf, vorzugsweise Heißdampf, bereitgestellt wird, das dem durch die Katalysatoreinrichtung (40) chemisch veränderten Behandlungsmittel zugemischt wird, wodurch das Behandlungsmittel verdünnt, vorzugsweise temperiert, wird, und das Gemisch auf das Verpackungsmittel (2) abgegeben wird.

15. Verfahren nach einem der Ansprüche 8 bis 14, **dadurch gekennzeichnet, dass** das Verpackungsmittel (2) ein Behälter ist und das durch die Katalysatoreinrichtung (40) chemisch veränderte Behandlungsmittel in das Behälterinnere eingebracht, vorzugsweise eingesprüht, wird.

## Claims

1. Device (1) for treating packaging means (2), in particular for sterilising containers in a beverage filling plant, using a treatment agent, wherein the device (1) has:
a treatment body (30) which is configured to impinge the packaging means (2) at least in some portions with the treatment agent;
a treatment agent supply line (10) which is configured to supply the treatment agent to the treatment body (30); and
a catalyst device (40) which is arranged in the treatment agent supply line (10) and is configured to be flowed through by the treatment agent and to alter the treatment agent chemically;
**characterised in that**
the treatment agent is a mixture of peracetic acid, acetic acid and hydrogen peroxide and the catalyst device (40) is configured to reduce the hydrogen peroxide component in the mixture.

2. Device (1) according to claim 1, **characterised in that** the catalyst device (40) has a catalyst chamber (41) filled with a catalytic material (42), preferably in the form of beads, which is inert with respect to the treatment agent, wherein the catalytic material (42) preferably comprises platinum oxide and/or manganese dioxide.

3. Device (1) according to claim 2, **characterised in that** the catalyst device (40) has a filter (43), preferably comprising a filter nonwoven, which is mounted in the catalyst chamber (41) so as to be able to be flowed through by the treatment agent.

4. Device (1) according to any of the preceding claims, **characterised in that** furthermore a steam supply line (20) is provided which is configured to supply steam, preferably hot steam, to the treatment body (30).

5. Device (1) according to claim 4, **characterised in that** the treatment body (30) has a mixing space (31) in fluid connection with the treatment agent supply line (10) and the steam supply line (20), which is configured to dilute the treatment agent using the steam, preferably to temper it, and to output the mixture onto the packaging means (2) via a nozzle.

6. Device (1) according to any of the preceding claims, **characterised in that** the treatment agent is present in the ratio of a stable equilibrium state.

7. Device (1) according to claim 6, **characterised in that** the catalyst device (40) is configured to reduce the proportion of hydrogen peroxide in the mixture.

8. Method for treating packaging means (2), in particular for sterilising containers in a beverage filling plant, using a treatment agent, wherein the method comprises:
providing a treatment agent, preferably sterilisation agent;
passing the treatment agent through a catalyst device (40), as a result of which the treatment agent is chemically changed;
impinging the packaging means (2) with the treatment agent which has been chemically changed by the catalyst device (40);
**characterised in that**
the treatment agent is a mixture of peracetic acid, acetic acid and hydrogen peroxide and the catalyst device reduces the proportion of hydrogen peroxide in the mixture.

9. Method according to claim 8, **characterised in that** the treatment agent consists of multiple components and the catalyst device (40) changes the proportion of one or more components.

10. Method according to claim 9, **characterised in that** the proportions of the components are in a chemical equilibrium state and the catalyst device (40) brings the treatment agent out of the chemical equilibrium state.

11. Method according to any of claims 8 to 10, **characterised in that** the catalyst device (40) has a catalyst chamber (41) filled with a catalytic material (42), preferably in the form of beads, which is inert with respect to the treatment agent, wherein the catalytic material (42) preferably comprises platinum oxide and/or manganese dioxide.

12. Method according to any of claims 8 to 11, **characterised in that** the treatment agent is present in the ratio of a stable equilibrium state.

13. Method according to claim 12, **characterised in that** the catalyst device (40) reduces the proportion of hydrogen peroxide from the mixture.

14. Method according to any of claims 8 to 13, **characterised in that** furthermore steam, preferably hot steam, is provided which is mixed into the treatment agent which has been chemically changed by the catalyst device (40), as a result of which the treatment agent is diluted, preferably tempered, and the mixture is outputted onto the packaging means (2).

15. Method according to any of claims 8 to 14, **characterised in that** the packaging means (2) is a container and the treatment agent which is chemically changed by the catalyst device (40) is introduced into the container interior, preferably is sprayed in.

## Revendications

1. Dispositif (1) pour le traitement de moyens d'emballage (2), en particulier pour la stérilisation de récipients dans une installation de remplissage de boissons, avec un agent de traitement, dans lequel le dispositif (1) comprend :
un organe de traitement (30) qui est agencé pour soumettre le moyen d'emballage (2) au moins par sections à l'agent de traitement ;
une conduite d'amenée d'agent de traitement (10) qui est agencée pour amener l'agent de traitement à l'organe de traitement (30) ; et
un dispositif catalyseur (40) disposé et agencé dans la conduite d'amenée d'agent de traitement (10) pour être parcouru par l'agent de traitement et pour modifier chimiquement l'agent de traitement ;
**caractérisé en ce que**
l'agent de traitement est un mélange d'acide peracétique, d'acide acétique et de peroxyde d'hydrogène et le dispositif catalyseur (40) est agencé pour réduire la teneur en peroxyde d'hydrogène dans le mélange.

2. Dispositif (1) selon la revendication 1, **caractérisé en ce que** le dispositif catalyseur (40) comporte une chambre de catalyseur (41) remplie d'un matériau catalyseur (42), de préférence sous la forme de billes, qui est inerte vis-à-vis de l'agent de traitement, dans lequel le matériau catalyseur (42) comprend de préférence de l'oxyde de platine et/ou du dioxyde de manganèse.

3. Dispositif (1) selon la revendication 2, **caractérisé en ce que** le dispositif catalyseur (40) comporte un filtre (43), de préférence comprenant un voile filtrant, qui est appliqué dans la chambre de catalyseur (41) en pouvant être parcouru par l'agent de traitement.

4. Dispositif (1) selon l'une quelconque des revendications précédentes, **caractérisé en ce qu'**une conduite d'amenée de vapeur (20) est de plus prévue, laquelle est agencée pour amener de la vapeur, de préférence de la vapeur surchauffée, à l'organe de traitement (30).

5. Dispositif (1) selon la revendication 4, **caractérisé en ce que** l'organe de traitement (30) comporte une chambre de mélange (31) en connexion fluidique avec la conduite d'amenée d'agent de traitement (10) et la conduite d'amenée de vapeur (20) qui est agencée pour diluer l'agent de traitement par la vapeur, de préférence pour tempérer, et diffuser le mélange via une buse sur le moyen d'emballage (2).

6. Dispositif (1) selon l'une quelconque des revendications précédentes, **caractérisé en ce que** l'agent de traitement se trouve dans un rapport d'état d'équilibre stable.

7. Dispositif (1) selon la revendication 6, **caractérisé en ce que** le dispositif catalyseur (40) est agencé pour réduire la teneur en peroxyde d'hydrogène dans le mélange.

8. Procédé pour le traitement de moyens d'emballage (2), en particulier pour la stérilisation de récipients dans une installation de remplissage de boissons, avec un agent de traitement, dans lequel le procédé (1) comprend les étapes suivantes :
fourniture d'un agent de traitement, de préférence d'un agent de stérilisation ;
passage de l'agent de traitement à travers un dispositif catalyseur (40), ce qui permet à l'agent de traitement d'être modifié chimiquement ;
chargement du moyen d'emballage (2) en agent de traitement chimiquement modifié à travers le dispositif catalyseur (40) ;
**caractérisé en ce que**
l'agent de traitement est un mélange d'acide peracétique, d'acide acétique et de peroxyde d'hydrogène et le dispositif catalyseur réduit la teneur en peroxyde d'hydrogène dans le mélange.

9. Procédé selon la revendication 8, **caractérisé en ce que** l'agent de traitement comprend plusieurs composants et le dispositif catalyseur (40) modifie la part d'un ou de plusieurs composants.

10. Procédé selon la revendication 9, **caractérisé en ce que** les parts des composants se trouvent dans un état d'équilibre chimique et **en ce que** le dispositif catalyseur (40) déstabilise l'état d'équilibre chimique de l'agent de traitement.

11. Procédé selon l'une quelconque des revendications 8 à 10, **caractérisé en ce que** le dispositif catalyseur (40) comporte une chambre de catalyseur (41) remplie d'un matériau catalyseur (42), de préférence sous la forme de billes, qui est inerte vis-à-vis de l'agent de traitement, dans lequel le matériau catalyseur (42) comprend de préférence de l'oxyde de platine et/ou du dioxyde de manganèse.

12. Procédé selon l'une quelconque des revendications 8 à 11, **caractérisé en ce que** l'agent de traitement se trouve dans un rapport d'état d'équilibre stable.

13. Procédé selon la revendication 12, **caractérisé en ce que** le dispositif catalyseur (40) réduit la teneur en peroxyde d'hydrogène du mélange.

14. Procédé selon l'une quelconque des revendications 8 à 13, **caractérisé en ce que** de la vapeur, de préférence de la vapeur surchauffée, est en outre fournie, laquelle est mélangée à l'agent de traitement modifié chimiquement par le dispositif catalyseur (40), ce qui permet à l'agent de traitement d'être dilué, de préférence tempéré, et le mélange est diffusé sur le moyen d'emballage (2).

15. Procédé selon l'une quelconque des revendications 8 à 14, **caractérisé en ce que** le moyen d'emballage (2) est un récipient et l'agent de traitement modifié chimiquement par le dispositif catalyseur (40) est déposé, de préférence injecté, à l'intérieur du récipient.
